# EUROPÄISCHE PATENTSCHRIFT

(11) **EP 0 530 667 B1**
(45) Veröffentlichungstag und Bekanntmachung des Hinweises auf die Patenterteilung: **20.12.1995**
(21) Anmeldenummer: 92114567.8
(22) Anmeldetag: 27.08.1992
(51) Int. Cl.: A61M 5/148

(54) **Vorrichtung zum Entleeren von flexiblen Flüssigkeitsbehältern**
Device for emptying flexible fluid pouches
Dispositif pour vider les poches de fluide flexibles

(30) Priorität: 03.09.1991 DE 4129271
(43) Veröffentlichungstag der Anmeldung: 10.03.1993
(73) Patentinhaber: Fresenius AG, 61350 Bad Homburg (DE)
(72) Erfinder: Weber, Wolfram, W-6683 Spiesen-Elversberg (DE); Neumann, Hans-Jürgen, W-6690 St. Wendel-Niederkirchen (DE); Scherer, Thomas, W-6630 Saarlouis (DE); Gläser, Bernhard, W-6695 Tholey (DE)
(74) Vertreter: Fuchs Mehler Weiss

(56) Entgegenhaltungen:
- FR-A- 2 102 412
- US-A- 4 157 771
- US-A- 4 504 267

## Beschreibung

Die Erfindung geht aus von einer Vorrichtung zum Entleeren von flexiblen Flüssigkeitsbehältern gemäß dem Oberbegriff des Patentanspruchs 1, wie sie aus der US-A-4,157,771 bekannt ist, und eignet sich besonders zum Entleeren von Blutbeuteln in einem extrakorporalen Kreislauf in einer Single-Needle-Anordnung.

Die Single-Needle-Technik ist ein seit langem bekannter Bestandteil des Verfahrens zur schonenden Blutbehandlung. Dabei geschieht die Entnahme und Rückführung des zu reinigenden bzw. gereinigten Blutes durch eine einzige Nadel, d.h. durch eine einzige Punktionsstelle. Dieses Verfahren ist gefäßschonend und für den Patienten mit weniger Schmerzen verbunden.

Eine wesentliche Voraussetzung für die effiziente Dialysebehandlung mittels Single-Needle-Technik ist, daß die Entnahme und Rückführung von Blut zügig durchgeführt wird, so daß die Gesamtdauer der Behandlung nicht unzumutbar erhöht wird.

Ein zeitbestimmender Faktor bei der Dialysebehandlung mittels Single-Needle-Technik ist die Rückführung des gereinigten Blutes. Üblicherweise wird das gereinigte Blut in einem flexiblen Behälter gesammelt, um dann, wenn der arterielle Teil des Blutkreislaufs verriegelt ist, aus diesem Behälter zurück durch die Nadel in den Blutkreislauf des Patienten geführt zu werden. Das Entleeren dieses Behälters soll dabei möglichst schnell und mit gleichmäßigem Flüssigkeitsstrom erfolgen.

Weiterhin ist für eine effiziente Dialysebehandlung ein möglichst konstanter Druck im gesamten Dialysesystem erforderlich, um beispielsweise die behandelte Blutmenge genau zu erfassen, so daß Medikamente richtig zudosiert werden können und Pumpenleistungen richtig eingestellt werden können.

Bekannte Vorrichtungen, die zu diesem Zweck verwendet werden, sind beispielsweise nachstehenden Ausführungen zu entnehmen.

In dem US-Patent US 4,991,743 wird eine Vorrichtung beansprucht, bei der es möglich ist, den auf dem flexiblen Flüssigkeitsbehälter ausgeübten Druck einzustellen. Es handelt sich hierbei um eine Platte, die durch Scherenheber mit Druck beaufschlagt wird, und die dann diesen Druck über die gesamte Entnahmephase vollflächig auf den flexiblen Flüssigkeitsbeutel weiterleitet. Diese Art der Druckregelung berücksichtigt nicht die veränderlichen Druckverhältnisse im flexiblen Flüssigkeitsbeutel während der Entnahme, denn aus der Patentschrift ist kein Hinweis darauf zu entnehmen, daß der Druck den veränderlichen Druck- und Volumenverhältnissen des Beutels angepaßt wird.

In der deutschen Offenlegungsschrift DE 39 11 587 A1 wird eine Vorrichtung beschrieben, bei der ein flexibler Beutel zwischen zwei Andruckplatten angeordnet ist, von denen mindestens eine beweglich und antreibbar ist. Diese Vorrichtung erweist sich insofern als nachteilig, als eine aufwendige Druckmeßvorrichtung an den Andruckplatten installiert sein muß, und eine umfangreiche Kontrolle erforderlich ist.

In der französischen Patentschrift FR-A-2102412 wird eine Vorrichtung zum Zusammendrücken eines flexiblen Beutels zwischen zwei Platten beschrieben, bei der die erste Platte ortsfest und die zweite Platte beweglich an der ortsfesten ersten Platte angelenkt ist und gegen die ortsfeste Platte verschwenkt werden kann. Als nachteilig ist bei dieser bekannten Vorrichtung anzusehen, daß sie ebenfalls nicht die veränderlichen Druckverhältnisse während der Entnahme berücksichtigt.

In dem eingangs erwähnten US-Patent US-A-4,157,771 wird eine Vorrichtung beschrieben, bei der ein flexibler Beutel zwischen zwei Platten angeordnet ist, von denen die erste Platte ortsfest und die zweite Platte gegenüber der erste Platte beweglich angeordnet ist. Weiterhin weist diese Vorrichtung ein Kraftelement und Hebelsystem als Kraftübertragungsmittel auf, das mit der zweiten Platte verbunden ist und die vom Kraftelement eingeleitete Kraft so umsetzt, daß die Kraft auf die bewegliche Platte während des Entleerungsvorganges zunimmt. Als nachteilig ist bei dieser bekannten Vorrichtung die aufwendige und viele Einzelteile aufweisende Konstruktion anzusehen, die sowohl bei der Herstellung als auch bei der Montage hohe Kosten verursacht. Weiterhin ist durch die Säulenführung der beweglichen Platte leicht ein Verkanten der beweglichen Platte und damit ein Klemmen der gesamten Vorrichtung möglich, so daß die Betriebssicherheit dieser Vorrichtung nicht gewährleistet ist.

Die zitierten Schriften zeigen, daß es bisher nicht auf einfache Art und Weise möglich war, unerwünschte stetige Druckänderungen während der Entnahme von gereinigtem Blut aus einem flexiblen Behälter zu vermeiden.

Somit liegen bei der Rückführung von gereinigtem Blut mittels Single-Needle-Technik oft ungleichmäßige Druckverhältnisse im Dialysesystem vor, die zu schwer kontrollierbaren Behandlungsdauern führen. Zudem verlängert sich die Behandlungsdauer außerordentlich, da, besonders gegen Ende der Entnahme von Blut aus dem flexiblen Behälter, bedingt durch den abnehmenden Druck, eine stetig abnehmende Flüssigkeitsmenge je Zeiteinheit befördert wird.

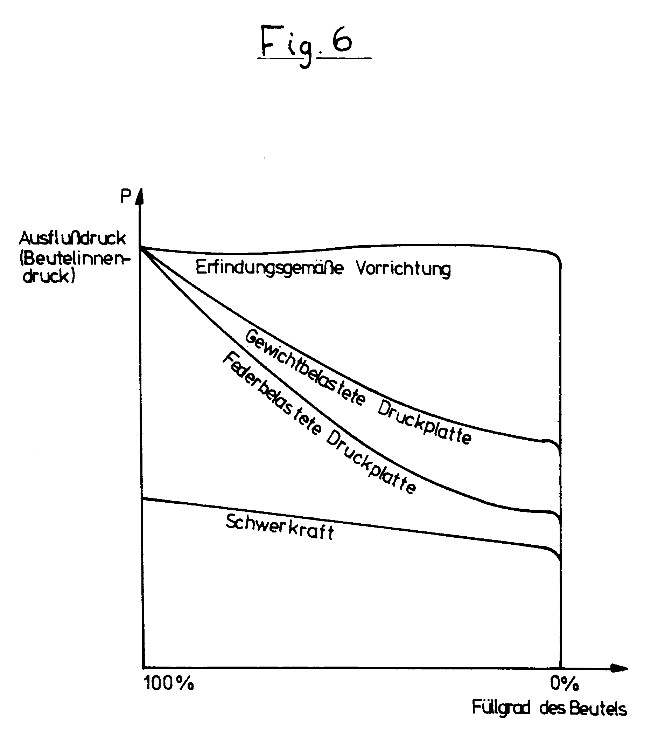

Besonders deutlich wird die Problematik des Druckabfalls in Figur 6 dargestellt. Die in der US-Patentschrift 4,991,743 beanspruchte Vorrichtung weist beispielsweise die Charakteristika einer quasi gewichtsbelasteten Druckplatte auf. Bei dieser Vorrichtung beträgt der Druckabfall gegen Ende der Blutentnahme aus dem flexiblen Behälter ca. 50 %, bezogen auf den Ausgangsdruck, da die gleichbleibende Kraft sich auf die stetig zunehmende Oberfläche des Beutels, die an den Andruckplatten anliegt, verteilt, und somit der Druck (Kraft / Fläche) abnimmt. Bedingt durch diesen Druckverlauf sind signifikante Druckschwankungen im Dialysekreislauf sowie eine erhebliche Verlängerung der Blutrückführungsphase zum Patienten (abnehmender Druck bedingt abnehmende Strömungsgeschwindigkeit) unvermeidbar.

Vor diesem Hintergrund ist es daher Aufgabe der Erfindung, eine einfache Vorrichtung zur Entleerung eines flexiblen Flüssigkeitsbehälters bereitzustellen, mit Hilfe derer dieser mit möglichst konstanter und möglichst hoher Geschwindigkeit vollständig entleert werden kann.

Diese Aufgabe wird bei einer Vorrichtung der eingangs erwähnten Art durch die Merkmale des kennzeichnenden Teils des Anspruchs 1 gelöst.

Die Vorrichtung wird in der Weise betrieben, daß die auf den flexiblen Behälter aufgebrachte Kraft durch Kraftübertragungsmittel in ihrem Betrag so verändert wird, daß der flexible Flüssigkeitsbehälter sowohl in gefülltem als auch in nahezu entleertem Zustand mit nahezu konstantem Druck beaufschlagt wird.

Dazu wirkt die betragsmäßig veränderte Kraft auf eine bewegbare Platte, die mit einer ortsfesten Platte verbunden ist und auf den zwischen der ortsfesten und der bewegbaren Platte angeordneten flexiblen Behälter.

Eine derartige Anordnung gewährleistet ein über die gesamte Entnahmephase nahezu konstantes Ausflußvolumen aus dem flexiblen Behälter, mit der Folge, daß einerseits die Druckverhältnisse im Dialysesystem nahezu konstant gehalten werden können, und daß andererseits die Zeit, die für die Reinfusion des gereinigten Blutes erforderlich ist, deutlich verkürzt werden kann, so daß die gesamte Behandlungsdauer des Dialysepatienten minimiert werden kann.

Weiterhin bedarf diese Vorrichtung nicht aufwendiger, komplexer und teurer Kontrollgeräte, da sie in der Konstruktion einfach ist und somit eine zuverlässige Funktion gewährleistet und zudem damit in der Herstellung und der Montage kostengünstig ist.

Bei einer bevorzugten Ausführungsform wird als Kraftelement eine Gasdruckfeder eingesetzt, die mittels zweier durch den Kolben der Gasdruckfeder betätigter Hebel, die eine bevorzugte Ausführungsform des Kraftübertragungssystems darstellen, Druck auf die bewegbare Platte, die einseitig an die ortsfeste Platte angelenkt ist, ausübt. Während der Blutentnahmephase wird auf den flexiblen Behälter, der zwischen der ortsfesten und der angelenkten Platte angeordent ist, stetig Druck ausgeübt durch die Kolbenstange der Gasdruckfeder.

Der Druck wird durch den Hebelmechanismus auf die angelenkte Platte und von der Platte auf den flexiblen Behälter Übertragen.

Bei einer vorteilhaften Ausgestaltung der Erfindung kann die Gasdruckfeder mit dem daran befindlichen Hebelmechanismus für den Fall arretiert werden, daß der flexible Behälter aus dem Raum zwischen der ortsfesten und der angelenkten Platte entfernt werden soll, beispielsweise nach beenderter Dialyse. Das Arretieren geschieht durch Umlegen eines Hebels, der an dem Ende des Kraftelements angebracht ist, das den Kraftübertragungsmitteln gegenüberliegt. Kraftelement und Kraftübertragungsmittel werden dabei so weit angehoben, daß auch bei fortgesetzter Übertragung des Drucks keine Kraft mehr auf den flexiblen Behälter ausgeübt wird.

Bei einer weiteren Ausführungsform können die Kraftübertragungsmittel in der Weise ausgestaltet sein, daß die ortsfeste und die angelenkte Platte durch eine Scherenführung oder eine Parallelführung verbunden sind. Weiterhin können in anderen Ausführungsformen die Kraftübertragungsmittel zwischen ortsfester und angelenkter Platte als Parallelogrammlenker oder aber auch als Säulenlenker ausgeformt sein.

In weiteren vorteilhaften Ausgestaltungen können als Kraftelemente verschiedenste Federn verwendet werden, wobei Federn mit annähernd konstanten Federkraft bevorzugt werden.

Eine weitere vorteilhafte Ausgestaltung weist zwei Kraftübertragungssysteme auf, die jeweils als Scherenführung ausgebildet sind und die durch ein Kraftelement mit Kraft beaufschlagt werden.

Eine weitere vorteilhafte Ausführung der Vorrichtung weist ein Gehäuse auf, das mit einer Öffnung zur Entnahme des flexiblen Behälters versehen ist, wobei die ortsfeste Platte den Boden des Gehäuses bildet.

Bei einer anderen vorteilhaften Ausführungsform ist der flexible Flüssigkeitsbehälter in der Weise zwischen der ortsfesten und der bewegbaren Platte angeordnet, daß seine Öffnung den aneinander angelenkten Plattenenden gegenüberliegt.

Die Erfindung wird nun anhand der folgenden Ausführungsbeispiele gemäß der Zeichnungen 1 bis 5 näher erläutert.

Hierbei zeigt:
- Figur 1: Eine schematische Darstellung einer Vorrichtung zum Entleeren eines flexiblen Flüssigkeitsbehälters, in der Stellung, in der ein nicht dargestellter Flüssigkeitsbehälter entleert ist.
- Figur 2: Eine schematische Darstellung einer Vorrichtung zum Entleeren von flexiblen Flüssigkeitsbehältern, gemäß Fig. 1, in der Stellung, in der ein nicht dargestellter Flüssigkeitsbehälter gefüllt ist.
- Figur 3: Eine schematische Darstellung einer Vorrichtung zum Entleeren von flexiblen Flüssigkeitsbehältern mit einer Arretiervorrichtung, wobei die Arretiervorrichtung geöffnet ist.
- Figur 4: Eine schematische Darstellung einer Vorrichtung zum Entleeren von flexiblen Flüssigkeitsbehältern mit einer Arretiervorrichtung, gemäß Fig. 3, wobei die Arretiervorrichtung verriegelt ist.
- Figur 5: Eine schematische Darstellung einer Vorrichtung zum Entleeren von flexiblen Flüssigkeitsbehältern, wobei die ortsfeste Platte und die bewegbare Platte durch eine Scherenführung verbunden sind.
- Figur 6: Ein Diagramm zur Darstellung des Ausflußdruckes des flexiblen Behälters in Abhängigkeit vom Füllgrad des Behälters in bekannten Vorrichtungen im Vergleich zur erfindungsgemäßen Vorrichtung.

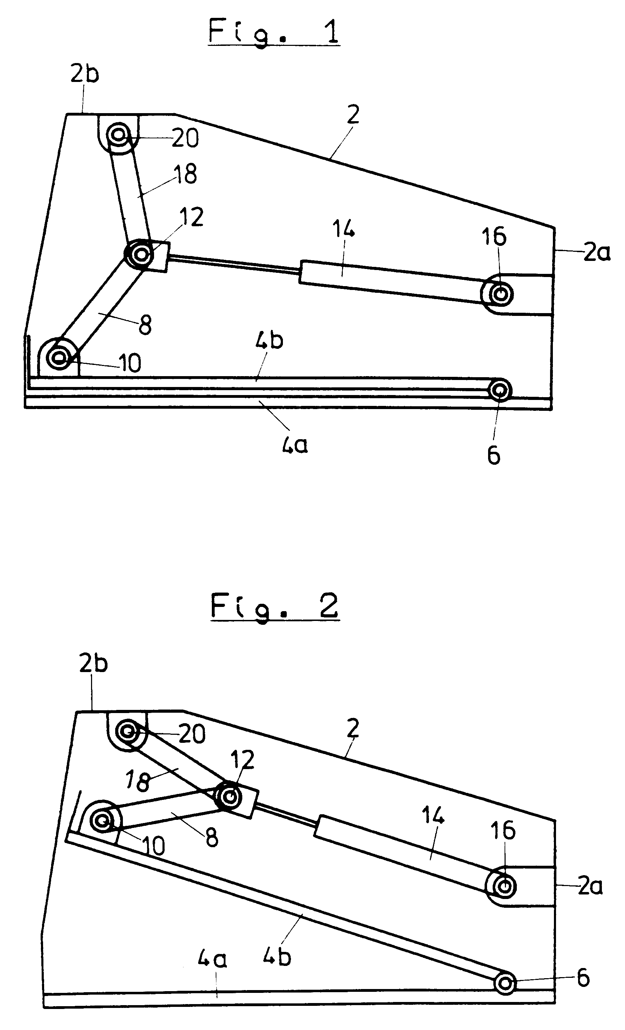

In Figur 1 ist eine bevorzugte Ausführungsform der Erfindung schematisch dargestellt. Im Gehäuse 2 sind Kraftelement, bewegbare 4b und ortsfeste 4a Platte, Kraftübertragungsmittel 8, 18 und flexibler Flüssigkeitsbehälter (nicht dargestellt) angeordnet.

Im Gehäuse 2 befinden sich zunächst eine ortsfeste Platte 4a und eine bewegbare Platte 4b, die um ein Gelenk 6 herum relativ zu der ortsfesten Platte 4a in einem Bereich von 0 bis maximal 90 ° verschwenkbar ist, wobei ein Schwenkbereich von 0 bis 25° bevorzugt wird.

An der bewegbaren Platte 4b ist ein erster Hebelarm 8 befestigt, der in seiner Halterung um ein Gelenk 10 drehbar ist. Das andere Ende des Hebelarms 8 ist verbunden mit einem Gelenkpunkt 12. An dem Gelenkpunkt 12 greift das Kraftelement 14 an, das in diesem Fall als Gasdruckfeder ausgebildet ist. Das dem Gelenkpunkt 12 gegenüberliegende Ende des Kraftelementes ist durch ein Gelenk 16 an der Gehäuseinnenwand 2a befestigt. Am Gelenkpunkt 12 greift weiterhin ein zweiter Hebelarm 18 an, der am Gelenkpunkt 12 ebenfalls drehbar gelagert ist, und dessen dem Gelenkpunkt 12 gegenüberliegendes Ende durch ein Gelenk 20 an der Gehäuseoberwand 2b befestigt ist.

Kraftelement und Kraftübertragungsmittel wirken in der Weise zusammen, daß beim Entleeren des Behälters durch das Kraftelement 14 mittels eines Gasdruckzylinderkolbens auf den Gelenkpunkt 12 Kraft ausgeübt wird. Mittels der aufgebrachten Kraft werden die Hebelarme 8 und 18 aus ihrer Ruhestellung herausbewegt und drücken dabei die bewegbare Platte 4b in Richtung auf die ortsfeste Platte 4a. Dadurch wird der hier nicht dargestellte flexible Flüssigkeitsbehälter mit Druck beaufschlagt. Die Kraft, die das Krafelement 14 auf die Kraftübertragungsmittel 8, 18 ausübt, und die von diesen auf die bewegbare Platte, und damit auf den flexiblen Flüssigkeitsbehälter übertragen wird, verändert sich in Abhängigkeit vom Füllgrad des Flüssigkeitsbehälters, indem mit abnehmendem Füllgrad die vom Kraftelement 14 auf die Kraftübertragungsmittel 8 und 18 ausgeübte Kraft aufgrund der kraftverstärkenden Hebelwirkung dieser Kraftübertragungsmittel einen gleichbleibenden Druck auf den flexiblen Flüssigkeitsbehälter ausübt. Die durch das Kraftelement 14 bewegten Hebelarme 8 und 18 werden, unabhängig von der auf sie ausgeübten Kraft, in einem Winkel zwischen mindestens 0 und höchstens 180°, vorzugsweise aber von 25 bis 75° zueinander verschwenkt.

In Figur 2 ist diesselbe Auführungsform wie in Figur 1, jedoch bei gefülltem Behälter (hier nicht dargestellt) abgebildet. Der Behälter wird mittels einer Pumpe gegen den Druck des Kraftelementes befüllt. Bei gefülltem Behälter wird die durch das gespannte Kraftelement auf den Gelenkpunkt 12 ausgeübte Kraft durch eine anfangs geringe Auslenkung der Hebelarme 8 und 18 nur wenig in ihrem Betrag verändert. Der Winkel zwischen den Hebeln 8 und 18 ist jetzt kleiner als bei nahezu vollständig entleertem Behälter (s. Figur 1).

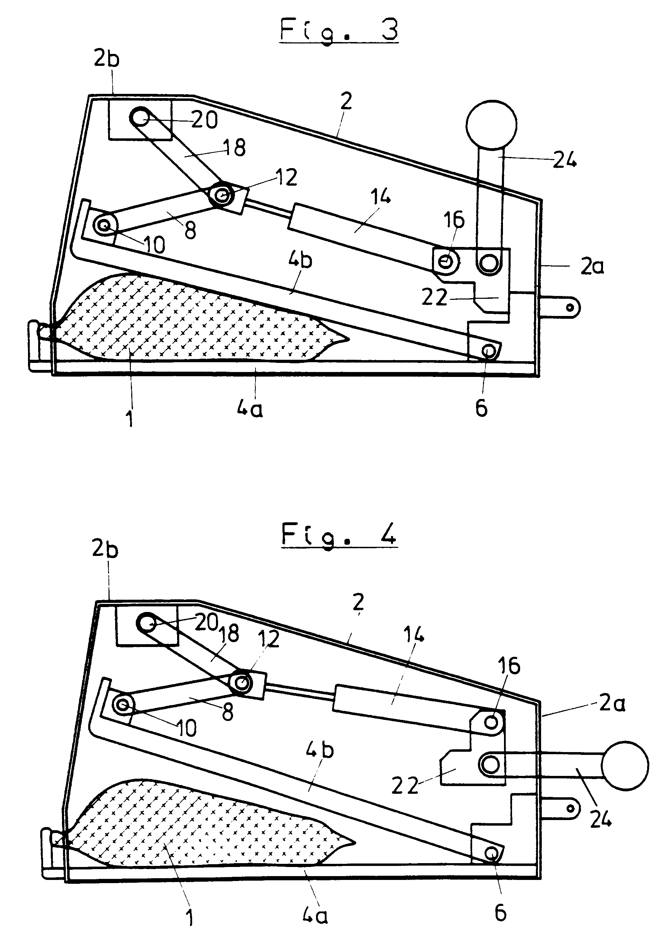

Die in Figur 3 dargestellte Ausführungsform der vorliegenden Erfindung weist Kraftelemente, Platten und Kraftübertragungsmittel auf, die mit den in Figuren 1 und 2 dargestellten Kraftelementen, Platten und Kraftübertragungsmitteln identisch sind. Hinsichtlich der Wirkungsweise dieser Elemente wird daher auf die vorstehenden Ausführungen verwiesen.

Zusätzlich zu den in Figuren 1 und 2 dargestellten Elementen weist die in Figur 3 dargestellte Ausführungsform eine Arretierungsvorrichtung, die nicht verriegelt ist, für das Kraftelement 14 und die Kraftübertragungsmittel 8, 18 auf. Die Arretierungshebel 24, mit dem die Arretierungsvorrichtung betätigt wird, sowie aus einem weiteren Hebel 22, der bei Betätigen des Arretierungshebels 24 Kraftelement und Kraftübertragungsmittel so aus ihrer Ausgangslage herausschwenkt, daß sie auf die bewegbare Platte 4b, und damit auf den flexiblen Flüssigkeitsbehälter 1, keinen Druck mehr ausüben könne.

Fig. 4 zeigt die gleiche Arretierungsvorrichtung in verriegeltem Zustand. Durch Betätigen des Arretierungshebels 24 wurden Kraftelement und Kraftübertragungsmittel so weit aus ihrer Ursprungslage herausgeschwenkt, daß die Gasdruckfeder 14 keine Kraft auf die Hebelarme 8 und 18 ausübt und die bewegbare Platte 4b nicht mehr weit genug ausgelenkt werden würde, um auf den flexiblen Flüssigkeitsbehälter 1 Druck auszuüben.

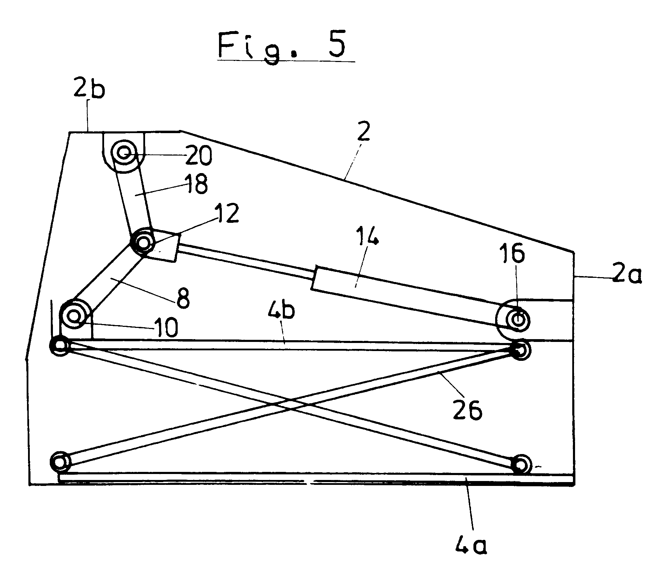

Figur 5 zeigt eine weitere Ausführungsform der vorliegenden Erfindung. Im Unterschied zu den in Figuren 1 - 4 dargestellten Ausführungsformen sind die ortsfeste Platte 4a und die bewegbare Platte 4b hier durch eine Scherenführung 26 verbunden.

Andere Ausführungsformen (hier nicht dargestellt) können mehrere Scherenführungen zwischen der ortsfesten und der bewegbaren Platte aufweisen, oder beide Platten können durch eine oder mehrere Parallelführungen verbunden sein.

Figur 6 sind die Kennlinien für den Ausflußdruck bei verschiedenen Entleerungsvorrichtungen dargestellt. Bei einer Entleerung des flexiblen Füssigkeitsbehälters mittels Schwerkraft ist zwar ein geringer Druckabfall zwischen hohem und und niedrigem Füllgrad des Behälters festzustellen, jedoch ist der Gesamtdruck niedrig, so daß sich nur eine geringe Ausflußrate einstellt. Zusätzlich durch feder- oder gewichtsbelastete Druckplatten beschwerte Flüssigkeitsbehälter können einen höheren Ausflußdruck bei gefülltem Behälter aufnehmen, jedoch sinkt mit abnehmendem Füllgrad des Behälters der Druck - und damit die Ausflußgeschwindigkeit - stark ab. Bei der erfindungsgemäßen Vorrichtung, die einen an einem Hebel angreifenden Federmechanismus beinhaltet, kann ein erhöhter Ausflußdruck bei vollem Behälter aufgebracht werden, der bis zur vollständigen Entleerung nahezu konstant gehalten werden kann.

## Patentansprüche

1. Vorrichtung zum Entleeren von flexiblen Flüssigkeitsbehältern,
insbesondere von Blutbeuteln in einem extrakorporalen Kreislauf in einer Single-Needle-Anordnung, mit einer ortsfesten Platte (4a), einer gegenüber der ortsfesten Platte beweglich angeordneten zweiten Platte (4b), wobei die beiden Platten (4a,4b) zur Aufnahme des flexiblen Flüssigkeitsbehälters (1) ausgebildet sind, einem Kraftelement (14) und Kraftübertragungsmitteln (8,18), die mit der zweiten Platte (4b) verbunden sind, wobei die Kraftübertragungsmittel (8,18) die vom Kraftelement (14) in sie eingeleitete Kraft so umsetzen, daß die auf die bewegbare Platte (4b) wirkende Kraft während des Entleerungsvorgangs zunimmt, bei der die Kraftübertragungsmittel (8,18) ein Hebelsystem bilden,
dadurch gekennzeichnet,
daß sie in einem Gehäuse (2) angeordnet ist, dessen Grundplatte die ortsfeste Platte (4a) bildet, daß das Kraftelement (14) mit einem Ende an einem Lagerpunkt (16) an der Gehäuseinnenwand angebracht und mit seinem anderen Ende in einem Gelenkpunkt (12) mit den Kraftübertragungsmitteln (8,18) verbunden ist, daß die Kraftübertragungsmittel (8,18) Kräfte auf die bewegbare Platte (4b) und den flexiblen Flüssigkeitsbehälter (1) übertragen und daß das Gehäuse eine Öffnung aufweist.

2. Vorrichtung nach Anspruch 1, dadurch gekennzeichnet, da das Kraftelement (14) eine Feder, insbesondere eine Gasdruckfeder, ist.

3. Vorrichtung nach Anspruch 1 oder 2, dadurch gekennzeichnet, daß die Feder mit ihrer Schubstange in einem Gelenkpunkt (12) an den Enden zweier, ein Hebelsystem bildender Hebelarme (8, 18) angeordnet ist, von denen ein erster Hebelarm (8) mit seinem anderen Ende an die bewegbare Platte (4b) angelenkt und der zweite Hebelarm (18) mit seinem anderen Ende an der Gehäuseoberwand (2b) angelenkt ist.

4. Vorrichtung nach Anspruch 3, dadurch gekennzeichnet, daß die beiden Hebelarme (8, 18) beim Entlastungsvorgang und beim Spannungsvorgang der Feder einen Winkel zwischen 0 und 180°, vorzugsweise 25 bis 75°, überstreichen.

5. Vorrichtung nach Anspruch 1, dadurch gekennzeichnet, daß der Lagerpunkt (16) ein an einer Gehäuseinnenseitenwand (2a) liegender Fixpunkt ist.

6. Vorrichtung nach Anspruch 1, dadurch gekennzeichnet, daß der Lagerpunkt (16) an einem Kipphebel (22) liegt, durch den, mittels eines dritten Hebelarms (24), das Kraftelement (14) und die Kraftübertragungsmittel (8, 18) sowie die zweite Platte (4b) aus ihrer jeweiligen Arbeitsposition ausrückbar sind.

7. Vorrichtung nach Anspruch 1, dadurch gekennzeichnet, daß die ortsfeste Platte (4a) und die bewegbare Platte (4b) durch eine oder mehrere Scherenführungen (26) verbunden sind.

8. Vorrichtung nach Anspruch 1, dadurch gekennzeichnet, daß die ortsfeste Platte (4a) und die bewegbare Platte (4b) durch eine oder mehrere Parallelführungen verbunden sind.

9. Vorrichtung nach Anspruch 1, dadurch gekennzeichnet, daß das Kraftelement (14) zwischen zwei Kraftübertragungsmitteln angeordnet ist, auf die es Kraft überträgt.

## Claims

1. A device for draining flexible fluid containers, in particular blood bags in an extracorporeal system of a single needle arrangement, having a stationary plate (4a), a second plate (4b) movably disposed opposite said stationary plate, with both plates (4a, 4b) being configured to receive said flexible fluid container, a force member (14) and force transfer means (8, 18) connected to said second plate (4b), with the force transfer means (8, 18) transferring the force received by it from said force member (14) in such a way that the force acting on said movable plate (4b) increases during the draining operation, where the force transfer means (8, 18) form a lever system,
characterized in
that they are disposed in a housing (2) whose base plate forms said stationary plate (4a), that said force member (14) is mounted with one end to a bearing point (16) at the interior wall of said housing and with its other end is connected at a lunge point (12) to said force transfer means (8, 18), that said force transfer means (8, 18) transfer forces to said movable plate (4b) and to said flexible fluid container (1) and that said housing comprises an opening.

2. The device according to claim 1, characterized in that said force member (14) is a spring, in particular a gas pressure spring.

3. The device according to claim 1 or 2, characterized in that said spring is mounted with its push rod to a hinge point (12) at the ends of two lever arms (8, 18) forming a lever system, the first lever arm (8) of which being hingeably attached at its other end to said movable plate (4b) and the second lever arm of which being hingeably attached at its other end to the upper wall (2b) of said housing.

4. The device according to claim 3, characterized in that both lever arms (8, 18) during the draining operation and the tensing operation of the spring sweep an angle of between 0 and 180°, preferably 25 to 75°.

5. The device according to claim 1, characterized in that said bearing point (16) is a fixed point located on an inner side wall (2a) of said housing.

6. The device according to claim 1, characterized in that said bearing point (16) is disposed on a tipping lever (22) with which the force member (14) and the force transfer means (8, 18) as well as said second plate (4b) are moved by means of a third lever arm (24) from their respective operating position.

7. The device according to claim 1, characterized in that said stationary plate (4a) and said movable plate (4b) are linked to one another by one or more scissors guides (26).

8. The device according to claim 1, characterized in that said stationary plate (4a) and said movable plate (4b) are linked to one another by multiple parallel guides.

9. The device according to claim 1, characterized in that said force member (14) is disposed between two force transfer means upon which it transfers force.

## Revendications

1. Dispositif de vidange de conteneurs de liquide souples, en particulier de poches de sang dans un circuit extracorporel selon un montage à simple aiguille et comportant
une plaque fixe (4a), une deuxième plaque (4b) montée mobile par rapport à la plaque fixe, les deux plaques (4a, 4b) étant conçues pour recevoir le conteneur souple (1),
un élément de force (14) et des moyens (8, 18) de transmission de force, qui sont reliés à la deuxième plaque (4b), les moyens (8, 18) de transmission de force transformant la force qu'ils reçoivent de l'élément de force (14) de manière à ce que la force agissant sur la plaque mobile (4b) augmente pendant le processus de vidange, grâce aux moyens (8, 18) de transmission de force constituant un système à levier,
caractérisé en ce que le dispositif est monté dans un boitier (2), dont la plaque de base constitue la plaque fixe (4a),
en ce que l'élément de force (14) est monté par une extrémité sur un point formant palier (16) sur la paroi intérieure du boîtier, et est relié par son autre extrémité avec les moyens (8, 18) de transmission de force par un point d'articulation (12),
en ce que les moyens (8, 18) de transmission de force transmettent des forces à la plaque mobile (4a) et au conteneur souple (1)
et en ce que le boîtier présente une ouverture.

2. Dispositif selon la revendication 1, caractérisé en ce que l'élément de force (14) est un ressort, en particulier un ressort à gaz sous pression.

3. Dispositif selon la revendication 1 ou 2, caractérisé en ce que le ressort est monté par un point d'articulation (12) de sa tige d'entraînement, à l'extrémité de deux bras de levier (8, 18) formant un système de leviers, parmi lesquels un premier bras de levier (8) est articulé par son autre extrémité sur la plaque mobile (4b) et le deuxième bras de levier (18) est articulé par son autre extrémité sur la paroi supérieure du boîtier (2b).

4. Dispositif selon la revendication 3, caractérisé en ce que lors du processus de détente et lors du processus de tension du ressort les deux bras de levier (8, 18) décrivent un angle compris entre 0 et 180°, de préférence entre 25 et 75°.

5. Dispositif selon la revendication 1, caractérisé en ce que le point d'articulation (16) est un point fixe situé sur une paroi latérale intérieure (2a) du boîtier.

6. Dispositif selon la revendication 1, caractérisé en ce que le point d'articulation (16) est situé sur un levier basculant (22), par lequel au moyen d'un troisième bras de levier (24), l'élément de force (14) et les moyens (8, 18) de transmission de force ainsi que la deuxième plaque (4b) peuvent quitter leurs positions de travail respectives.

7. Dispositif selon la revendication 1, caractérisé en ce que la plaque fixe (4a) et la plaque mobile (4b) sont reliées par un ou plusieurs guidages en ciseaux (26).

8. Dispositif selon la revendication 1, caractérisé en ce que la plaque fixe (4a) et la plaque mobile (4b) sont reliées par un ou plusieurs guidages parallèles.

9. Dispositif selon la revendication 1, caractérisé en ce que l'élément de force (14) est monté entre les deux moyens de transmission de force, auxquels il transmet une force.
